# EUROPÄISCHE PATENTSCHRIFT

(11) **EP 3 879 259 B1**
(45) Veröffentlichungstag und Bekanntmachung des Hinweises auf die Patenterteilung: **15.11.2023**
(21) Anmeldenummer: 21159375.1
(22) Anmeldetag: 25.02.2021
(51) Int. Cl.: G01N 21/64, C12Q 1/686

(54) **ANORDNUNG UND VERFAHREN ZUR PCR MIT MEHRKANALIGER FLUORESZENZMESSUNG FÜR RÄUMLICH VERTEILTE PROBEN**
SYSTEM AND METHOD FOR PCR WITH MULTI-CHANNEL FLUORESCENCE MEASUREMENT FOR SPATIALLY DISTRIBUTED SAMPLES
AGENCEMENT ET PROCÉDÉ DESTINÉS À LA PCR COMPRENANT LA MESURE DE FLUORESCENCE À CANAUX MULTIPLES POUR ÉCHANTILLONS SPATIALEMENT DISTRIBUÉS

(30) Priorität: 12.03.2020 DE 102020106865
(43) Veröffentlichungstag der Anmeldung: 15.09.2021
(73) Patentinhaber: Analytik Jena GmbH+Co. KG, 07745 Jena (DE)
(72) Erfinder: Winter, Stefan, 07774 Dornburg-Camburg (DE)
(74) Vertreter: Koslowski, Christine Adelheid

(56) Entgegenhaltungen:
- DE-A1-102006 036 171
- US-A1- 2012 190 034
- US-A1- 2014 045 186

## Beschreibung

Die vorliegende Erfindung betrifft eine Anordnung zur Polymerase-Kettenreaktion (engl. polymer chain reaction (PCR)) mit mehrkanaliger Fluoreszenzmessung sowie ein entsprechendes Verfahren.

Die PCR ist ein weit verbreitetes Analyseverfahren im Bereich der Biotechnologie und Molekularbiologie und ermöglicht, beispielsweise durch Verwendung geeigneter Thermocycler, eine Vielzahl von Proben, insbesondere gleichzeitig, zu untersuchen. Dabei werden als Probenträger in der Regel Mikrotiterplatten eingesetzt, bei denen eine Vielzahl von Proben in einem Probenträger angeordnet werden kann.

Thermocycler bieten die Möglichkeit, thermisch kontrollierte Prozessschritte mit unterschiedlichen Temperaturzyklen selbstständig und automatisiert durchzuführen. Darüber hinaus sind sogenannte real-time Thermocycler bekannt geworden, welche zur Durchführung einer quantitativen Echtzeit PCR dienen. Solche Geräte sind mit zusätzlichen Optikmodulen zur Fluoreszenzmessung ausgestattet. Dies ermöglicht eine Quantifizierung der erhaltenen Reaktionsprodukte in Echtzeit. Zur Fluoreszenzmessung werden geeignete Fluoreszenzfarbstoffe eingesetzt, die bei Entstehung des gewünschten Reaktionsprodukts eine verstärkte Fluoreszenz anzeigen, oder die an speziellen Sonden endständig gebunden sind und während der gewünschten Reaktion jeweils so verändert werden, dass verstärkte Fluoreszenz auftritt. Die Fluoreszenzmessungen werden dann üblicherweise nach jedem Temperaturzyklus in allen zu untersuchenden Proben in Phasen konstanter Temperatur durchgeführt und beispielsweise graphisch dargestellt. Dabei können auch jeweils unterschiedliche Farbstoffe und/oder Sonden verwendet werden. Eine Unterscheidung kann in diesem Falle anhand der unterschiedlichen Emissionsspektren erfolgen. Dies wird auch als Fluoreszenz-Multiplexing bezeichnet.

Aus dem Stand der Technik sind verschiedenste unterschiedliche Vorrichtungen zur Durchführung einer real-time PCR bekannt geworden, welche die Detektion von Fluoreszenz bei einer oder mehreren unterschiedlichen Anrege- und Emissionswellenlängen erlauben. Je nach verwendeter Vorrichtung wird dazu beispielsweise eine simultane Beobachtung sämtlicher Proben einer zweidimensionalen Anordnung von Proben, z.B. mittels eines bildgebenden Verfahrens, oder ein Proben-Multiplexing realisiert.

Zur Bewältigung immer höherer Probenaufkommen werden die Thermocycler derart optimiert, dass die notwendige Anzahl an Temperaturzyklen in möglichst kurzer Zeit bewältigbar sind. Dies bedeutet, dass die Aufheiz- und Abkühlphasen, sowie die Phasen konstanter Temperatur immer weiter verkürzt werden (sog. Rapid PCR). Hierdurch werden aber auch die Zeitintervalle zur Durchführung von Fluoreszenzmessungen immer kürzer. So sind aus der DE102006036171A1 eine Anordnung und ein Verfahren zur schnellen, mehrkanaligen Fluoreszenzmessung in PCR Proben bekannt geworden, welche/welches innerhalb einer kurzen Zeit in einer Vielzahl von Proben die Messung der Fluoreszenz in mehreren Farbkanälen ermöglicht. Durch eine rotierende Anordnung der Optikmodule wird eine schnelle Umschaltung der einzelnen Wellenlängen gewährleistet. Probenseitig kommt ein Objektscanner zum Einsatz, mittels welchem für eine Vielzahl von Proben nacheinander die Fluoreszenzmessungen durchgeführt werden können, welche darüber hinaus mit dem jeweiligen Temperaturzyklus synchronisiert werden. Mit einer derartigen Anordnung lässt dich allerdings jeweils nur ein Probenträger in Form einer Mikrotiterplatte analysieren, welche nur einen Temperaturzyklus durchlaufen kann.

Die Anmeldung US 2012/0190034 A1 zeigt eine Vorrichtung zur optischen Auslesung von Mikrotiterplatten. Die DE 10 2006 036 171 A1 beschreibt eine Anordnung zur schnellen PCR mit mehrkanaliger Fluoreszenzmessung. Die US 2014/0045186 A1 offenbart einen Thermocycler zur Untersuchung von zwei mikrofluidischen Kartuschen.

Deshalb liegt der vorliegenden Erfindung die Aufgabe zugrunde, eine schnelle Fluoreszenzmessung auch für solche Geräte zu ermöglichen, welche mehrere unabhängige Temperaturzyklen durchlaufen können.

Diese Aufgabe wird gelöst durch die Anordnung nach Anspruch 1 sowie durch das Verfahren nach Anspruch 9.

Hinsichtlich der Anordnung wird die der Erfindung zugrundeliegende Aufgabe gelöst durch eine Anordnung zur PCR mit mehrkanaliger Fluoreszenzmessung umfassend
- zwei Heizkörper mit jeweils einer Probenaufnahme zur Aufnahme je eines Probenträgers, wobei die Probenträger jeweils eine Vielzahl an in Reihen und Spalten angeordneten Kavitäten aufweisen,
- zwei Messköpfe, welche jeweils relativ zu den Heizkörpern beweglich sind, wobei jeder Messkopf derart ausgestaltet ist, dass mittels des Messkopfes Anregungslicht in jede der Kavitäten in einer Spalte des jeweiligen Probenträgers einkoppelbar und Fluoreszenzlicht aus den Kavitäten in der Spalte auskoppelbar ist, und
- eine Fluoreszenz-Einheit mit

einem Träger in Form einer rotierbaren Scheibe,
zumindest einem zumindest teilweise auf dem Träger angeordneten Optikmodul zur Erzeugung von Anregungslicht mit zumindest einer vorgebbaren Wellenlänge und einer Detektionseinheit, zur Erfassung einer Fluoreszenz in den Kavitäten, und
einem Koppelmodul, welches jeweils eine Aufnahme für jeweils zumindest einen Lichtleiter zur Leitung des Anregungslichts von der Fluoreszenz-Einheit zu den Messköpfen und zur Leitung des Fluoreszenzlichts von den Messköpfen zur Fluoreszenz-Einheit aufweist.

Die Probenaufnahmen bilden also jeweils eine zweidimensionale Anordnung zur Aufnahme einer Vielzahl von Proben, welche beispielsweise auf einem Probenträger in Form einer Mikrotiterplatte mit einer Vielzahl an Kavitäten zur Aufnahme jeweils einer Probe angeordnet sein können. Die Messköpfe sind beispielsweise lateral und/oder vertikal zum jeweiligen Probenträger beweglich und dienen jeweils der Abtastung je eines Probenträgers. Beispielsweise kann jeder der Messköpfe nacheinander über die verschiedenen Spalten der zweidimensionalen Anordnung - also Reihe für Reihe - bewegt werden.

Bei dem Optikmodul handelt es sich insbesondere um eine zumindest teilweise austauschbare Baugruppe. Das Optikmodul weist beispielsweise eine Lichtquelle, verschiedene Filter, wie einen Fluoreszenzanregungsfilter und/oder einen Fluoreszenzemissionsfilter, Linsen, und/oder Strahlteiler auf. In diesem Zusammenhang sei auf die aus dem Stand der Technik in vielen unterschiedlichen Ausgestaltungen bekannten Optikmodule verwiesen, welche allesamt auch für die vorliegende Erfindung verwendet werden können. Auch bezüglich der Detektionseinheit sei auf die aus dem Stand der Technik an sich bekannten Varianten verwiesen. Beispielsweise handelt es sich bei der Detektionseinheit um einen Photomultiplier. Bei dem Lichtleiter wiederum kann es sich beispielsweise um eine Lichtleitfaser oder ein Faserbündel handeln. Die Messköpfe verfügen beispielsweise über eine mechanische Einheit mit einem Rahmen, welcher eine Trägerplatte aufweist, und über einen in Führungen beweglichen, insbesondere von einem Motor angetriebenen, Schlitten.

Erfindungsgemäß werden voneinander unabhängige Messköpfe für die unterschiedlichen Heizkörper mit den unterschiedlichen Probenträgern, welche unterschiedliche Temperaturzyklen durchlaufen können eingesetzt, welche alle mit einer gemeinsamen Fluoreszenzeinheit synchronisierbar sind. Die einzelnen Messköpfe können auch zeitlich unabhängig voneinander operieren. Dies reduziert den gerätetechnischen Aufwand erheblich, da nur eine kostenintensive Fluoreszenzeinheit notwendig ist.

In einer Ausgestaltung sind auf dem Träger zumindest zwei Optikmodule, insbesondere umfangsverteilt, entlang einer Kreisbahn auf der rotierenden Scheibe angeordnet. Auf diese Weise wird eine Fluoreszenzmessung mit mehreren Farbkanälen realisiert.

Eine weitere Ausgestaltung beinhaltet, dass die Fluoreszenz-Einheit eine Vorrichtung zur Bestimmung einer Umdrehungszahl des Trägers aufweist. Alternativ kann auch eine von der Umdrehungszahl ableitbare Größe ermittelt werden, wie beispielsweise eine Winkelgeschwindigkeit. Anhand der Umdrehungszahl ist es möglich, die relative Position der Scheibe, bzw. die jeweilige relative Position jedes der Optikmodule zu ermitteln.

In diesem Zusammenhang ist es von Vorteil, wenn die Vorrichtung einen Optokoppler und ein auf der Scheibe angeordnetes Schaltelement, insbesondere eine Schaltfahne, umfasst. Diese Anordnung ermöglicht auf besonders einfache Art und Weise eine Ermittlung der Umdrehungszahl.

In einer Ausgestaltung umfasst die Anordnung einen Temperatursensor zur Bestimmung und/oder Überwachung einer Temperatur von zumindest einem der Heizkörper und/oder Probenträger. Vorzugsweise ist für jeden der Heizkörper bzw. Probenträger jeweils zumindest ein Temperatursensor vorgesehen.

Schließlich beinhaltet noch eine Ausgestaltung der Anordnung, dass das Koppelmodul relativ zum Träger derart angeordnet ist, dass während einer vollständigen Umdrehung der Scheibe nacheinander von jedem auf dem Träger angeordneten Optikmodul Anregungslicht in jeden der Messköpfe und Fluoreszenzlicht von jedem Messkopf zu jedem Optikmodul gelangt. Das Koppelmodul ist demnach relativ zur rotierenden Scheibe derart angeordnet, dass es mit jedem der Optikmodule in Kontakt kommt, bzw. derart, dass mittels jedem der Optikmodule Anregungslicht in die Lichtleiter einkoppelbar ist.

In diesem Zusammenhang ist es einerseits denkbar, dass eine Länge des Koppelmoduls tangential zu der rotierenden Scheibe an einen durch die Kreisradien zweier benachbarter Optikmodule gebildeten Kreisbogen angepasst ist, insbesondere wobei die Länge kleiner ist als eine Kreissehne der Kreisradien zweier benachbarter Optikmodule. Eine derartige Anordnung vermeidet, dass mittels zwei unterschiedlicher Optikmodule zum gleichen Zeitpunkt Anregungslicht in die Koppeleinheit gelangt. Eine derartige Ausgestaltung ist insbesondere von Vorteil, wenn die Scheibe mit einer konstanten Umdrehungsgeschwindigkeit rotiert wird.

Andererseits kann das Koppelmodul auch radial zur rotierenden Scheibe beweglich ausgestaltet sein. In diesem Falle sind die rotierende Scheibe und das Koppelmodul beweglich.

Die der Erfindung zugrundeliegende Aufgabe wird ferner gelöst durch ein Verfahren zur PCR mit mehrkanaliger Fluoreszenzmessung, umfassend folgende Verfahrensschritte:
- Einkoppeln von Anregungslicht jeweils in Kavitäten einer Spalte einer zweidimensionalen Anordnung einer Vielzahl von Kavitäten von zumindest zwei Probenträgern mittels jeweils eines Messkopfes,
- Erfassen der Fluoreszenz aus den Kavitäten der jeweiligen Spalte des jeweiligen Probenträgers, und
- schrittweises laterales Bewegen von jedem der Messköpfe über die Spalten der zweidimensionalen Anordnung der Kavitäten jedes Probenträgers, um alle in der zweidimensionalen Anordnung der Kavitäten befindlichen Proben jedes Probenträgers zur Fluoreszenz anzuregen und die Fluoreszenz zu erfassen.

Es wird also Anregungslicht gleichzeitig in die zumindest zwei unterschiedlichen Messköpfe eingekoppelt, welche der Abtastung zumindest zweier unterschiedlicher Probenträger, welche auch unterschiedliche Temperaturzyklen durchlaufen können, dienen. Damit kann mittels einer einzigen Fluoreszenzeinheit eine Vielzahl unterschiedlicher Probenträger mit jeweils einer Vielzahl an Proben hinsichtlich der Fluoreszenz untersucht werden.

Das Verfahren findet bevorzugt Anwendung für eine erfindungsgemäße Anordnung nach zumindest einer der beschriebenen Ausgestaltungen.

Erfindungsgemäß wird eine schrittweise laterale Bewegung jedes Messkopfes in Abhängigkeit einer Rotationsgeschwindigkeit eines als rotierende Scheibe ausgestalteten Trägers, auf welchem zumindest ein Optikmodul, vorzugsweise zumindest zwei Optikmodule, zur Erzeugung des Anregungslichts angeordnet ist, gewählt. Die Optikmodule und die rotierende Scheibe sind Teil der Fluoreszenzeinheit. Die Proben, welche jeweils in einer Reihe des jeweiligen Probenträgers angeordnet sind, können so jeweils vollständig abgetastet werden, bevor innerhalb der Fluoreszenzeinheit zwischen zwei unterschiedlichen Optikmodulen gewechselt wird.

In diesem Zusammenhang ist es von Vorteil, wenn die schrittweise laterale Bewegung nach einer vollständigen Umdrehung der rotierenden Scheibe durchgeführt wird. Auf diese Weise wird sichergestellt, dass die jeweils untersuchte Reihe von Proben in jedem der Probenträger jeweils mittels allen Farbkanälen untersucht worden ist, bevor die jeweils folgende Reihe von Proben in jedem der Probenträger zur Fluoreszenz angeregt wird.

Es ist ferner von Vorteil, wenn eine Dauer der lateralen Bewegung von jedem der Messköpfe zwischen zwei aufeinander folgenden Reihen eine Dauer für eine vollständige Umdrehung der Scheibe entspricht. Auf diese Weise kann die laterale Bewegung der Messköpfe auf einfache Art und Weise mit der Rotationsgeschwindigkeit der rotierenden Scheibe synchronisiert werden.

In einer weiteren Ausgestaltung des Verfahrens wird überprüft, ob eine vorgegebene Temperatur eines Heizkörpers und/oder des Probenträgers erreicht ist, wobei eine erfasste Fluoreszenz nur aufgezeichnet wird, wenn die vorgegebene Temperatur erreicht wird. Auf diese Weise können Fehler bezüglich der Auswertung der gemessenen Fluoreszenzen vermieden werden, welche dadurch entstehen, dass im jeweils in einem Probenträger ablaufenden Temperaturzyklus ein bestimmter Sollwert für die Temperaturen der jeweiligen Proben noch nicht erreicht ist. Die Fluoreszenzmessung erfolgt bevorzugt in Phasen konstanter Temperatur während des jeweiligen Tem peraturzyklus.

In diesem Zusammenhang ist es wiederum von Vorteil, wenn die schrittweise laterale Bewegung eines Messkopfes und Aufzeichnung der erfassten Fluoreszenz erst nach Erreichen der vorgebbaren Temperatur durchgeführt wird.

Es sei darauf verwiesen, dass sich die in Zusammenhang mit der erfindungsgemäßen Anordnung beschriebenen Ausgestaltungen mutatis mutandis auch auf das erfindungsgemäße Verfahren anwenden lassen und umgekehrt.

Nähere Einzelheiten der Erfindung sowie ihrer vorteilhaften Ausgestaltungen werden anhand der nachfolgenden Figuren erläutert. Es zeigt:
Fig. 1: eine schematische Darstellung einer ersten möglichen Ausgestaltung einer erfindungsgemäßen Vorrichtung,
Fig. 2: eine schematische Darstellung einer erfindungsgemäßen Vorrichtung mit drei räumlich entkoppelten, unabhängigen Probenanordnungen, und
Fig. 3: eine schematische Darstellung einer erfindungsgemäßen Vorrichtung mit zwei unabhängigen Probenanordnungen und beweglichem Koppelmodul.

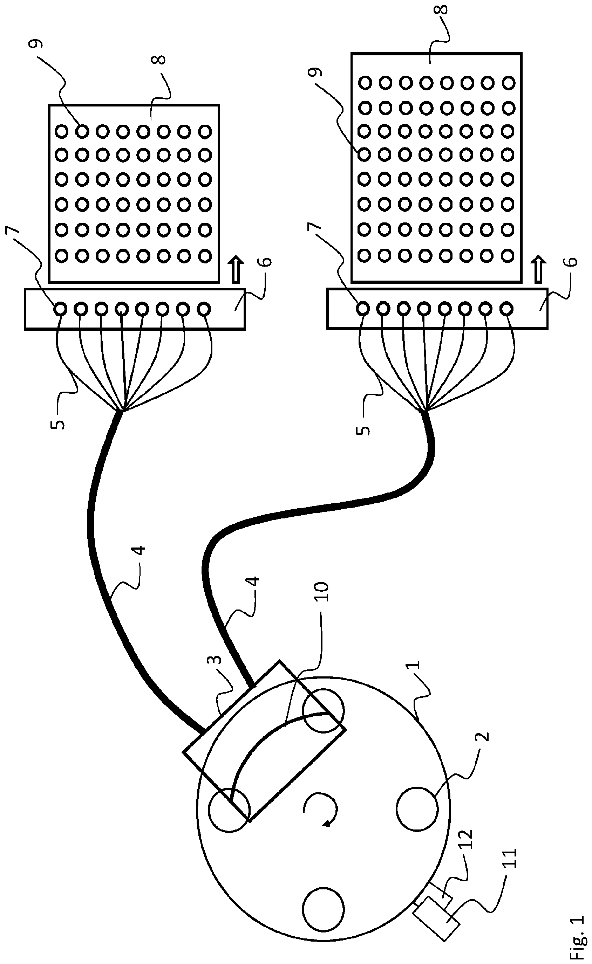

In Fig. 1 ist eine schematische Darstellung einer ersten möglichen Ausgestaltung einer erfindungsgemäßen Anordnung gezeigt. Die Anordnung weist hier exemplarisch zwei Heizkörper 8 auf, in welchen Probenträger bzw. Proben 9 jeweils in Reihen und Spalten angeordnet sind. Jedem Heizkörper 8 ist ferner je ein Messkopf 6 zugeordnet, welcher in Richtung des Pfeils über den jeweiligen Heizkörper 8 bewegbar ist. Über einzelne Fasern 5 und Linsen 6 wird in jede Probe 9 einer Spalte Anregungslicht eingekoppelt und Fluoreszenzlicht ausgekoppelt. Die Anzahl an Fasern 5 bzw. Linsen 6 in jedem der Messköpfe 6 entspricht der Anzahl an Proben 9 einer Reihe des jeweiligen Heizkörpers 8. Es sei darauf verwiesen, dass unterschiedliche Heizkörper 8 über eine unterschiedliche Anzahl an Probenaufnahmen und über unterschiedliche Aufteilungen der Proben über die Reihen und Spalten verfügen. Entsprechend können unterschiedliche Messköpfe 6 über unterschiedlich viele Fasern 5 verfügen.

Die Fasern 5 sind jeweils in einem dem Messkopf 6 abgewandten Bereich zu einem Lichtleiter 4 in Form eines Faserbündels zusammengefasst, welches zu einer einzigen, für sämtliche Messköpfe 6 verwendeten Fluoreszenz-Einheit geleitet wird. Die Lichtleiter 4 münden jeweils in ein Koppelmodul 3, welches für jeden der Lichtleiter 4 jeweils eine Aufnahme, welche entlang der Kreisbahn 10 angeordnet sind, aufweist. Auf einem Träger 1 sind vier verschiedene Optikmodule 2 umfangverteilt angeordnet, so dass bei einer Rotation der Scheibe 1 jedes der Optikmodule 2 nacheinander das Koppelmodul 3 passiert. Auf diese Weise kann gewährleistet werden, dass jedes Optikmodul 2 während einer vollständigen Umdrehung des Trägers 1 einzeln Anregungslicht zu allen Messköpfen 6 und Fluoreszenzlicht von allen Messköpfen 6 zur Fluoreszenzeinheit leiten kann. Die Länge des Koppelmoduls 3 tangential zu der rotierenden Scheibe 1 ist somit an die Abstände der einzelnen Optikmodule 2 angepasst. Insbesondere ist die Länge an einen durch die Kreisradien zweier benachbarter Optikmodule 2 gebildeten Kreisbogen 10 angepasst, hier so, dass die Länge kleiner ist als eine Kreissehne der Kreisradien zweier benachbarter Optikmodule 2.

Ferner ist auf dem Träger 1 eine Vorrichtung zur Bestimmung einer Umdrehungszahl des Trägers 1 mit einem Optokoppler 11 und einer Schaltfahne 12 angeordnet. Dadurch kann jeweils die relative Position eines jeden Optikmoduls 2 relativ zum Koppelmodul 3 ermittelt werden. Optional, hier jedoch nicht graphisch dargestellt, können die Heizkörper 8 über Temperatursensoren verfügen, um die Temperaturen der einzelnen Heizkörper 8 während der jeweiligen Temperaturzyklen zu erfassen. Vorzugsweise wird die Fluoreszenz der Proben nur in Zeitintervallen konstanter Probentemperatur erfasst. Unterschiedliche Heizkörper 8 können dabei unterschiedliche Temperaturzyklen durchlaufen.

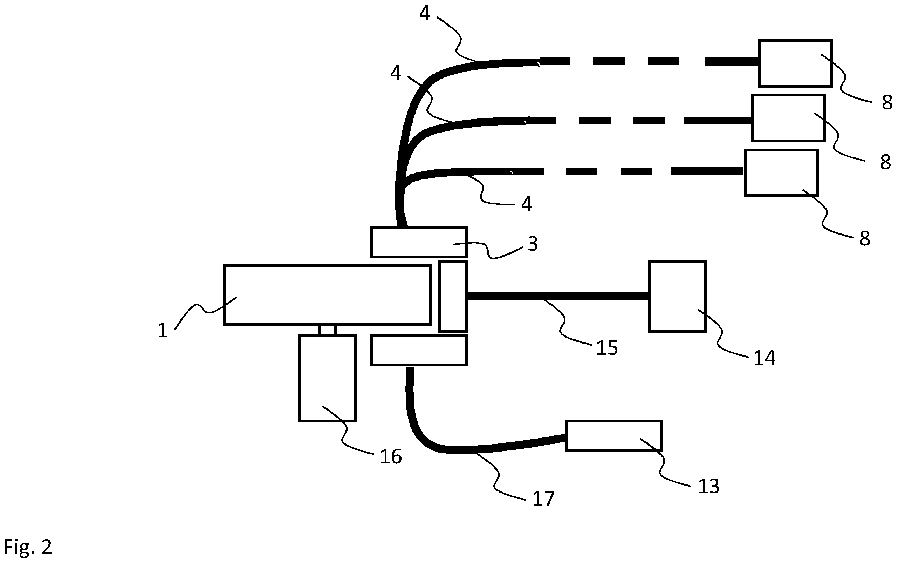

In Fig. 2 ist eine detaillierte Darstellung der Fluoreszenz-Einheit gezeigt. Die Einheit umfasst eine Lichtquelle 14, von welcher über einen Lichtleiter 15 in Form eines Faserbündels Anregungslicht in die einzelnen Messköpfe 6 eingekoppelt wird. Von den Proben 9 erfasstes Fluoreszenzlicht wird gleichzeitig über einen Lichtleiter 17 zu einer Detektionseinheit 13 geleitet und dort in elektrische Signale umgesetzt und zu einer hier nicht separat dargestellten Auswerteelektronik weitergeleitet.

Der Träger 1 wird für das hier gezeigte Beispiel mittels eines Motors 16 angetrieben und vorzugsweise mit konstanter Umdrehungsgeschwindigkeit rotiert. Nach einer vollständigen Umdrehung des Trägers 1 sind alle Proben 9 einer Spalte des Probenträgers durch alle vorhandenen Optikmodule 2 zur Fluoreszenz angeregt worden, so dass eine laterale Bewegung der Messköpfe 6 zur jeweils nächsten Spalte des Probenträgers erfolgen kann. In diesem Zusammenhang liefert die Vorrichtung 11,12 zur Bestimmung einer Umdrehungszahl der rotierenden Scheibe 1 ein Triggersignal, welches zur Synchronisierung der Abtastschritte bzw. der lateralen Bewegung der Messköpfe 6 und zur Zuordnung der jeweiligen zur Detektoreinheit 13 gelangenden Signale dient. Dieser Steuervorgang kann beispielsweise in einer ebenfalls hier nicht dargestellten Recheneinheit erfolgen.

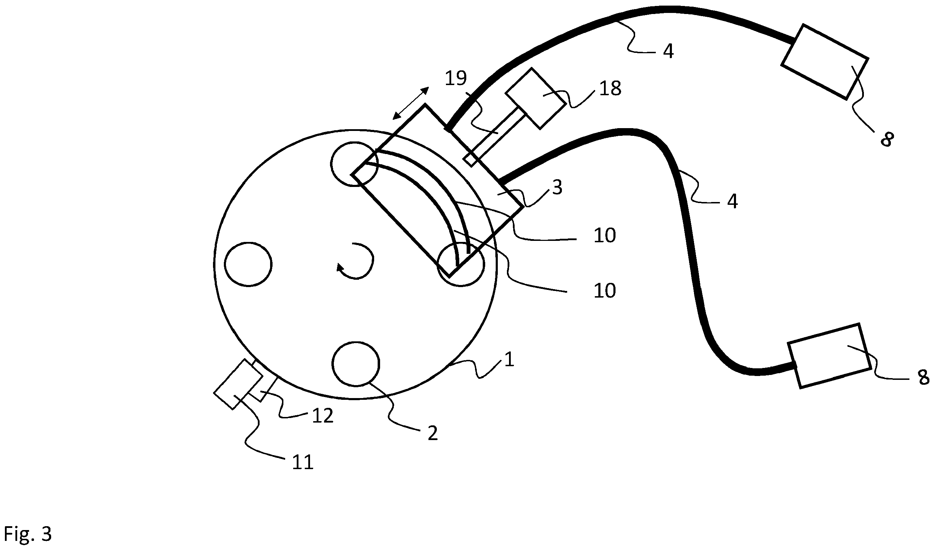

Eine zweite beispielhafte Ausgestaltung einer erfindungsgemäßen Anordnung ist in Fig. 3 skizziert. Die Fluoreszenz-Einheit ist so ausgestaltet, wie in Fig. 1 gezeigt. Im Unterschied zu Fig. 1 ist im Falle der Fig. 3 das Koppelmodul 3 radial zum Träger 1 in Richtung des eingezeichneten Pfeils beweglich. Die Bewegung kann, ähnlich wie im Falle des Trägers 1, durch einen Motor 18 bewerkstelligt werden.

### Bezugszeichen

- 1: Träger
- 2: Optikmodul
- 3: Koppelmodul
- 4: Faserbündel
- 5: Fasern
- 6: Messkopf
- 7: Linsen
- 8: Heizkörper
- 9: Probenträger, Probe
- 10: Kreisbogen
- 11: Optokoppler
- 12: Schaltfahne
- 13: Detektoreinheit
- 14: Lichtquelle
- 15: Faserbündel
- 16: Motor
- 17: Faserbündel
- 18: Motor

## Patentansprüche

1. Anordnung zur PCR mit mehrkanaliger Fluoreszenzmessung umfassend
- zwei Heizkörper (8) mit jeweils einer Probenaufnahme zur Aufnahme je eines Probenträgers (9), wobei die Probenträger (9) jeweils eine Vielzahl an in Reihen und Spalten angeordneten Kavitäten aufweisen,
- zwei Messköpfe (6), welche jeweils relativ zu den Heizkörpern (8) beweglich sind, wobei jeder Messkopf (6) derart ausgestaltet ist, dass mittels des Messkopfes (6) Anregungslicht in jede der Kavitäten in einer Spalte des jeweiligen Probenträgers (9) einkoppelbar und Fluoreszenzlicht aus den Kavitäten in der Spalte auskoppelbar ist, und
- eine Fluoreszenz-Einheit mit
einem Träger (1) in Form einer rotierbaren Scheibe, zumindest einem zumindest teilweise auf dem Träger (1) angeordneten Optikmodul (2) zur Erzeugung von Anregungslicht mit zumindest einer vorgebbaren Wellenlänge und einer Detektionseinheit (13) zur Erfassung einer Fluoreszenz in den Kavitäten, und
einem Koppelmodul (3), welches jeweils eine Aufnahme für jeweils zumindest einen Lichtleiter (4) zur Leitung des Anregungslichts von der Fluoreszenz-Einheit zu den Messköpfen (6) und zur Leitung des Fluoreszenzlichts von den Messköpfen (6) zur Fluoreszenz-Einheit aufweist.

2. Anordnung nach Anspruch 1,
wobei auf dem Träger (1) zumindest zwei Optikmodule (2), insbesondere umfangsverteilt, entlang einer Kreisbahn (10) auf der rotierenden Scheibe angeordnet sind.

3. Anordnung nach zumindest einem der vorherigen Ansprüche, wobei die Fluoreszenz-Einheit eine Vorrichtung (11,12) zur Bestimmung einer Umdrehungszahl des Trägers (1) aufweist.

4. Anordnung nach Anspruch 3,
wobei die Vorrichtung einen Optokoppler (11) und ein auf der Scheibe (1) angeordnetes Schaltelement (12), insbesondere eine Schaltfahne, umfasst.

5. Anordnung nach zumindest einem der vorherigen Ansprüche,
umfassend einen Temperatursensor zur Bestimmung und/oder Überwachung einer Temperatur von zumindest einem der Heizkörper und/oder Probenträger.

6. Anordnung nach zumindest einem der Ansprüche 2-5,
wobei das Koppelmodul (3) relativ zum Träger (1) derart angeordnet ist, dass während einer vollständigen Umdrehung der Scheibe (1) nacheinander von jedem auf dem Träger (1) angeordneten Optikmodul (2) Anregungslicht in jeden der Messköpfe (6) und Fluoreszenzlicht von jedem Messkopf (6) zu jedem Optikmodul (2) gelangt.

7. Anordnung nach Anspruch 6,
wobei eine Länge des Koppelmoduls (3) tangential zu der rotierenden Scheibe (1) an einen durch die Kreisradien zweier benachbarter Optikmodule (2) gebildeten Kreisbogen (10) angepasst ist, insbesondere wobei die Länge kleiner ist als eine Kreissehne der Kreisradien zweier benachbarter Optikmodule (2).

8. Anordnung nach Anspruch 6,
wobei das Koppelmodul (3) radial zur rotierenden Scheibe (1) beweglich ist.

9. Verfahren zur PCR mit mehrkanaliger Fluoreszenzmessung, umfassend folgende Verfahrensschritte:
- Einkoppeln von Anregungslicht jeweils in Kavitäten einer Spalte einer zweidimensionalen Anordnung einer Vielzahl von Kavitäten von zumindest zwei Probenträgern (9) mittels jeweils eines Messkopfes (6),
- Erfassen der Fluoreszenz aus den Kavitäten der jeweiligen Spalte des jeweiligen Probenträgers (9), und
- schrittweises laterales Bewegen von jedem der Messköpfe (6) über die Spalten der zweidimensionalen Anordnung der Kavitäten jedes Probenträgers (9), um alle in der zweidimensionalen Anordnung der Kavitäten befindlichen Proben jedes Probenträgers zur Fluoreszenz anzuregen und die Fluoreszenz zu erfassen, wobei eine schrittweise laterale Bewegung jedes Messkopfes (6) in Abhängigkeit einer Rotationsgeschwindigkeit eines als rotierende Scheibe ausgestalteten Trägers (1), auf welchem zumindest ein Optikmodul (2), vorzugsweise zumindest zwei Optikmodule (2), zur Erzeugung des Anregungslichts angeordnet ist, gewählt wird.

10. Verfahren nach Anspruch 9,
wobei die schrittweise laterale Bewegung nach einer vollständigen Umdrehung der rotierenden Scheibe (1) durchgeführt wird.

11. Verfahren nach einem der Ansprüche 9-10,
wobei überprüft wird, ob eine vorgegebene Temperatur eines Heizkörpers (8) und/oder des Probenträgers (9) erreicht ist, und wobei eine erfasste Fluoreszenz nur aufgezeichnet wird, wenn die vorgegebene Temperatur erreicht wird.

12. Verfahren nach Anspruch 11,
wobei die schrittweise laterale Bewegung eines Messkopfes (6) und Aufzeichnung der erfassten Fluoreszenz erst nach Erreichen der vorgebbaren Temperatur durchgeführt wird.

## Claims

1. An arrangement for PCR with multi-channel fluorescence measurement, comprising:
- Two heating elements (8), each with one sample mount for housing one sample carrier (9) in each case, wherein the sample carriers (9) each have a number of cavities arranged in rows and columns,
- Two measuring heads (6), which both move relative to heating elements (8), wherein each measuring head (6) is configured so that excitation light can be coupled into each of the cavities in a row on the respective sample carrier (9) using the measuring head (6) and fluorescent light can be coupled out of the cavities in the column, and
- a fluorescence unit with
a carrier (1) in the form of a rotating disk, at least one optical module (2) which is at least partly arranged on the carrier (1) for generating excitation light with at least one prespecifiable wavelength, and a detection unit (13) for detecting a fluorescence in the cavities, and
a coupling module (3), which has a mount for at least one light guide (4) in each case to guide the excitation light from the fluorescence unit to the measuring heads (6) and to guide the fluorescent light from the measuring heads (6) to the fluorescence unit.

2. The arrangement as claimed in claim 1,
wherein at least two optical modules (2), in particular ones distributed around the circumference, are arranged on the carrier (1) along a circular path (10) on the rotating disk.

3. The arrangement as claimed in at least one of the preceding claims, wherein the fluorescence unit has a device (11, 12) for determining a rotational speed of the carrier (1).

4. The arrangement as claimed in claim 3,
wherein the device comprises an optical coupler (11) and a switching element (12) arranged on the disk (1), in particular a switching lug.

5. The arrangement as claimed in at least one of the preceding claims, comprising a temperature sensor for detecting and/or monitoring a temperature of at least one of the heating elements and/or sample carriers.

6. The arrangement as claimed in at least one of claims 2 to 5,
wherein the coupling module (3) is arranged relative to the carrier (1) in such a way that during a full revolution of the disk (1), each optical module (2) arranged on the carrier (1) generates excitation light in each of the measuring heads (6) one after another, and fluorescent light is guided from each measuring head (6) to each optical module (2).

7. The arrangement as claimed in claim 6,
wherein a length of the coupling module (3) tangentially relative to the rotating disk (1) is adapted to an arc (10) formed by the radii of two adjacent optical modules (2), in particular wherein the length is smaller than a chord of the radii of two adjacent optical modules (2).

8. The arrangement as claimed in claim 6,
wherein the coupling module (3) moves radially relative to the rotating disk (1).

9. A method for PCR with multi-channel fluorescence measurement, comprising the following process steps:
- Coupling excitation light into each cavity in a column of a two-dimensional arrangement of a number of cavities on at least two sample carriers (9) using one measuring head (6) in each case,
- Detecting the fluorescence from the cavities in each column on each sample carrier (9), and
- Incremental lateral movement of each of the measuring heads (6) over the columns of the two-dimensional arrangement of the cavities on each sample carrier (9) in order to stimulate fluorescence in all samples located in the two-dimensional arrangement of the cavities on each sample carrier, and to detect said fluorescence, wherein an incremental lateral movement of each measuring head (6) is selected depending on a rotational speed of a carrier (1) configured as a rotating disk, on which at least one optical module (2), preferably at least two optical modules (2), is arranged to generate the excitation light.

10. The method as claimed in claim 9,
wherein the incremental lateral movement is performed after one complete revolution of the rotating disk (1).

11. The method as claimed in one of claims 9 to 10,
wherein a check is performed to establish whether a prespecified temperature of a heating element (8) and/or of the sample carrier (9) is reached, and wherein a detected fluorescence is only recorded if the prespecified temperature is reached.

12. The method as claimed in claim 11,
wherein the incremental lateral movement of a measuring head (6) and recording of the detected fluorescence are only performed once the prespecifiable temperature has been reached

## Revendications

1. Dispositif de réaction en chaîne avec mesure de fluorescence à plusieurs voies, lequel dispositif comprend :
- deux corps chauffants (8) comportant chacun un logement d'échantillon destiné à recevoir un porte-échantillon (9), les porte-échantillons (9) présentant chacun une pluralité de cavités disposées en rangées et en colonnes,
- deux têtes de mesure (6), lesquelles sont respectivement mobiles par rapport aux corps chauffants (8), chaque tête de mesure (6) étant conçue de telle sorte qu'au moyen de la tête de mesure (6), de la lumière d'excitation peut être injectée dans chacune des cavités d'une colonne du porte-échantillon (9) respectif et de la lumière fluorescente peut être extraite des cavités de la colonne, et
- une unité de fluorescence comprenant
un support (1) sous la forme d'un disque rotatif,
au moins un module optique (2) disposé au moins partiellement sur le support (1) et destiné à produire une lumière d'excitation avec au moins une longueur d'onde prédéfinissable et une unité de détection (13) destinée à détecter une fluorescence dans les cavités, et
un module de couplage (3), lequel présente respectivement un logement pour respectivement au moins un guide de lumière (4) pour la conduction de la lumière d'excitation de l'unité de fluorescence vers les têtes de mesure (6) et pour la conduction de la lumière de fluorescence des têtes de mesure (6) vers l'unité de fluorescence.

2. Dispositif selon la revendication 1,
pour lequel au moins deux modules optiques (2) sont disposés sur le support (1), notamment répartis sur la circonférence, le long d'une trajectoire circulaire (10) sur le disque rotatif.

3. Dispositif selon au moins l'une des revendications précédentes,
pour lequel l'unité de fluorescence comprend un dispositif (11, 12) destiné à la détermination d'un nombre de tours du support (1).

4. Dispositif selon la revendication 3,
lequel dispositif comprend un optocoupleur (11) et un élément de commutation (12), notamment une languette de commutation, lequel élément est disposé sur le disque (1).

5. Dispositif selon au moins l'une des revendications précédentes,
lequel dispositif comprend un capteur de température destiné à la détermination et/ou à la surveillance d'une température d'au moins l'un des corps chauffants et/ou porte-échantillons.

6. Dispositif selon au moins l'une des revendications 2 à 5,
pour lequel le module de couplage (3) est disposé par rapport au support (1) de telle sorte que, pendant une rotation complète du disque (1), de la lumière d'excitation passe successivement de chaque module optique (2) disposé sur le support (1) dans chacune des têtes de mesure (6) et de la lumière de fluorescence passe de chaque tête de mesure (6) vers chaque module optique (2).

7. Dispositif selon la revendication 6,
pour lequel une longueur du module de couplage (3) tangentielle au disque rotatif (1) est adaptée à un arc de cercle (10) formé par les rayons de cercle de deux modules optiques (2) voisins, notamment la longueur étant inférieure à une corde de cercle des rayons de cercle de deux modules optiques (2) voisins.

8. Dispositif selon la revendication 6,
pour lequel le module de couplage (3) est mobile radialement par rapport au disque rotatif (1).

9. Procédé de réaction en chaîne avec mesure de fluorescence à plusieurs voies, lequel procédé comprend les étapes suivantes :
- injection de lumière d'excitation respectivement dans les cavités d'une colonne d'un dispositif bidimensionnel d'une pluralité de cavités d'au moins deux porte-échantillons (9) au moyen d'une tête de mesure (6),
- Détection de la fluorescence issue des cavités de la colonne respective du porte-échantillon respectif (9), et
- Déplacement latéral pas à pas de chacune des têtes de mesure (6) sur les colonnes du dispositif bidimensionnel des cavités de chaque porte-échantillon (9), afin d'exciter en fluorescence tous les échantillons de chaque porte-échantillon situés dans le dispositif bidimensionnel des cavités et de détecter la fluorescence, un mouvement latéral pas à pas de chaque tête de mesure (6) étant choisi en fonction d'une vitesse de rotation d'un support (1) conçu comme un disque rotatif, support sur lequel est disposé au moins un module optique (2), de préférence au moins deux modules optiques (2), destiné(s) à produire la lumière d'excitation.

10. Procédé selon la revendication 9,
pour lequel le mouvement latéral pas à pas est effectué après une rotation complète du disque rotatif (1).

11. Procédé selon l'une des revendications 9 à 10,
pour lequel on vérifie si une température prédéterminée d'un corps chauffant (8) et/ou du porte-échantillon (9) est atteinte, et
pour lequel une fluorescence détectée n'est enregistrée que si la température prédéfinie est atteinte.

12. Procédé selon la revendication 11,
pour lequel le mouvement latéral pas à pas d'une tête de mesure (6) et l'enregistrement de la fluorescence détectée ne sont effectués qu'après avoir atteint la température prédéfinissable.
